# EUROPÄISCHE PATENTSCHRIFT

(11) **EP 2 288 318 B1**
(45) Veröffentlichungstag und Bekanntmachung des Hinweises auf die Patenterteilung: **06.11.2013**
(21) Anmeldenummer: 09728959.9
(22) Anmeldetag: 31.03.2009
(51) Int. Cl.: G02C 11/08, A61F 9/02

(54) **BRILLE MIT SEITENTEILEN MIT SCHUTZ- UND/ ODER SICHTFUNKTION**
EYE GLASSES HAVING SIDE PARTS COMPRISING A PROTECTIVE AND/OR VISION FUNCTION
LUNETTES MUNIES D'ÉLÉMENTS LATÉRAUX ASSURANT UNE FONCTION DE PROTECTION ET/OU RELATIVE À LA VISION

(30) Priorität: 31.03.2008 DE 102008016526; 07.07.2008 DE 102008031617
(43) Veröffentlichungstag der Anmeldung: 02.03.2011
(73) Patentinhaber: Schäfer, Klaus-Peter, 84036 Landshut (DE)
(72) Erfinder: Schäfer, Klaus-Peter, 84036 Landshut (DE)
(74) Vertreter: Kuhnen & Wacker
(86) Internationale Anmeldenummer: PCT/EP2009/002359
(87) Internationale Veröffentlichungsnummer: WO 2009/121573

(56) Entgegenhaltungen:
- WO-A-03/021334
- WO-A-2004/031839
- US-A1- 2003 035 082
- US-A1- 2004 174 492
- US-A1- 2005 088 613

## Beschreibung

Die vorliegende Erfindung betrifft eine Brille gemäß dem Oberbegriff des Anspruchs 1. Sie betrifft ferner Seitenteile zur Verwendung mit der erfindungsgemäßen Brille gemäß Anspruch 13 und ein Set gemäß Anspruch 14.

Aus der Praxis sind Brillen und insbesondere Schutzbrillen wie Arbeitsschutzbrillen bekannt, welche den Träger der Brille bei bestimmten Tätigkeiten wie der Metallbearbeitung oder dem Umgang mit gesundheitsgefährdenden Chemikalien und dergleichen vor Irritationen oder gar Verletzungen der Augen durch Späne, insbesondere glühende Späne, Spritzer von Säuren oder Basen, Licht jeglicher Wellenlänge oder Ursprungs oder dergleichen schützen.

Diese Brillen sind jedoch regelmäßig mit Nachteilen behaftet. So sind diese Brillen oft unbequem zu tragen und machen insbesondere Trägern von Korrekturbrillen Schwierigkeiten bei der gemeinsamen Verwendung von Schutzbrille und Korrekturbrille, weshalb die Schutzbrille regelmäßig auch in Situationen, für welche sie bestimmt ist, nicht aufgesetzt wird.

Aus der WO 02/074209 A1 geht eine Schutzbrille hervor, welche an unterschiedliche Trageumstände anpassbar ist. So kann die Brille durch das Anfügen eines inneren Rahmenteils auf ihrer Innenseite für Situationen umgerüstet werden, bei welchen der Träger der Brille einen besonderen Schutz seiner Augen wünscht. Ebenso kann die Brille aber auch ohne das innere Rahmenteil getragen werden. Dies ist insbesondere dann vorteilhaft, wenn die entsprechende Arbeit beziehungsweise Situation keinen besonderen Schutz der Augen erfordert oder das innere Rahmenteil als zugleich unnötig und störend empfunden würde.

Aus der US 2004/0174492 ist eine Brille bekannt, welche sich gleichermaßen zum Radfahren oder für Strandsportarten, als Sonnenbrille und als Schwimmbrille eignet.

Aufgabe der vorliegenden Erfindung ist es, eine weitere Brille, insbesondere eine weitere Schutzbrille, anzuheben.

Die erfindungsgemäße Aufgabe wird gelöst durch die Merkmalskombination des Anspruchs 1.

So wird erfindungsgemäß eine Brille, insbesondere eine Schutzbrille, vorgeschlagen, welche wenigstens ein Rahmenteil oder Gestell mit wenigstens einem Brillenglas und wenigstens eine erste Halteeinrichtung zum Halten der Brille beziehungsweise des Brillengestells am Kopf eines Trägers aufweist.

Die erfindungsgemäße Brille weist ferner eine Aufnahmeeinrichtung auf, welche ein oder zwei unabhängig voneinander vorliegende Seitenteile, welche die Brille aufweist, lösbar an der Brille, beispielsweise am Rahmenteil, aufnimmt. Das wenigstens eine Seitenteil weist dabei einen Funktionsabschnitt zum Schutz der Augen und/oder zum Verändern der Brillenglasfunktion auf. Der Funktionsabschnitt kann dabei einer Verringerung eines sich üblicherweise bei Trägern herkömmlicher Brillen zwischen Brille und Gesicht ergebenden Spalts dienen, durch welchen Partikel, Gase, Flüssigkeiten, Strahlen und dergleichen von der Seite her hinter das Brillenglas und damit in den Augenbereich gelangen können. Der Funktionsabschnitt kann jedoch auch die Brillenglasfunktion verändern, indem durch den Funktionsabschnitt durch dessen geeignete Ausgestaltung eine Verdunkelung bewirkt wird, welche bspw. gegen übermäßig Sonneneinwirkung, gegen Lichtentwicklung beim Schweißen, beim Umgang mit Lasern und dergleichen, bewirkt wird. Jede andere Funktionswirkung ist ebenfalls von der Erfindung umfasst. Dabei ist die Funktionswirkung als eine Schutzwirkung, eine Sichthilfe, eine Einrichtung zum Erhöhen des Trage- oder Sichtkomforts und dgl. zu verstehen. Ein Abschnitt, der eine solche Funktionswirkung erfüllt, fällt erfindungsgemäß unter den Begriff "Funktionsabschnitt".

Der Begriff "Seitenteil" umfasst erfindungsgemäß jede im Gebrauchszustand der Brille zwischen Rahmenteil und Gesicht des Brillenträgers vorgesehene, von der Brille trennbare Einrichtung mit den im Weiteren beschriebenen Eigenschaften. Das Seitenteil muss daher nicht seitlich - räumlich bezogen - auf einer anderen Struktur angeordnet sein.

Unter "Brille" wird erfindungsgemäß jede Art einer Brille, insbesondere eine Schutzbrille oder Arbeitsschutzbrille, verstanden. Da sie mit oder ohne Seitenteile verwendet werden kann, wird diese im Folgenden auch als eine Wechselbrille bezeichnet. Sie kann somit mit als auch ohne Seitenteile in zwei verschiedenen Zuständen verwendet werden, wobei ihre besondere Wirkung durch den Funktionsabschnitt beim Verwenden der Brille mit angebrachten Seitenteilen eintritt. Zudem kann dieselbe Brille ohne gleichzeitige Verwendung der Seitenteile ähnlich einer handelsüblichen Sonnen- oder Korrekturbrille ohne die Komfortbeeinträchtigung verwendet werden.

Unter dem Rahmenteil der Brille wird erfindungsgemäß ein Abschnitt verstanden, welcher das Brillenglas trägt oder in welchem das Brillenglas angeordnet ist. Unter "Rahmenteil" kann somit die Fassung des Brillenglases verstanden werden, ebenso auch der üblicherweise als "Gestell" bezeichnete Teil der Brille. Das Rahmenteil kann dabei geschlossen, halbgeschlossen oder mit nur abschnittsweisem Kontakt zum Brillenglas ausgestaltet sein.

Unter "Brillenglas" wird erfindungsgemäß jedes vom Rahmenteil ganz oder teilweise umfasste mehr oder weniger durchsichtige Material verstanden, durch welches hindurch der Träger einer Brille mit oder ohne Sichtkorrektur üblicherweise blickt. Das Brillenglas kann hierbei aus einem glashaltigen Material, einem Kunststoff oder dergleichen gefertigt sein. Es kann eine ebene Fläche bilden, gekrümmt sein, getönt sein, an verschiedene Lichtverhältnisse adaptierbar sein und dergleichen.

Unter der ersten Halteeinrichtung zum Halten der Brille am Kopf des Trägers wird erfindungsgemäß beispielsweise ein übliches Paar Brillenbügel verstanden. Es kann jedoch auch eine bandförmige Struktur zum Halten der Brille am Kopf des Trägers sein, welche den Kopf bandartig umgibt.

Die Aufnahmeeinrichtung, welche vorbereitet und geeignet ist zum reversiblen Aufnehmen von einem oder zwei unabhängigen Seitenteilen, ist derart ausgestaltet, dass der Benutzer der Brille diese durch Hinzufügen der Seitenteile an die Brille und durch deren Abnehmen von einem Benutzungszustand mit Seitenteilen in den anderen Benutzungszustand ohne Seitenteile - vorzugsweise ohne Einsatz von Werkzeugen allein mit seinen Händen - bewerkstelligen kann. Sie kann somit schnell an unterschiedliche Arbeitsumgebungen angepasst werden.

Das oder die Seitenteile können dabei - wie oben angemerkt - aufgrund ihres Funktionsabschnittes bspw. eine Verringerung oder gar Abdichtung eines bei herkömmlichen Brillen typischerweise auftretenden Spaltes zwischen Brille und Gesicht ermöglichen. Ziel eines solchen Funktionsabschnitts kann es sein, ein Eintreten von in Fest-, Gas- oder Flüssigzustand oder anderer Form wie Strahlung auftretenden schädlichen Effektoren, zum Beispiel Schweißlicht, Laserstrahl und dergleichen durch den Spalt und/ oder das Brillenglas, möglichst zuverlässig zu verhindern. Ist dabei nicht in jedem Fall ein Schutz gegen Eintritt des jeweiligen Effektors an jeder Stelle des Umfangs des jeweiligen Seitenteils erforderlich, so kann der Funktionsabschnitt beispielsweise nur in einem oberen Bereich des Seitenteils zum Schutz gegen von oben eintretenden Effektoren, in einem unteren Bereich des Seitenteils zum Schutz gegen von unten eintretenden Effektoren, usw. ausgestaltet bzw. vorgesehen sein. Jeder andere Funktionsabschnitt, welcher keinen Schutz gegen Eintreten von Schmutz durch den Spalt hindurch bieten soll, sondern eine andere Funktion erfüllt, ist erfindungsgemäße natürlich ebenfalls als Funktionsabschnitt zu verstehen und von der vorliegenden Erfindung umfasst.

Die mittels der erfindungsgemäßen Brille erzielbaren Vorteile umfassen unter anderem eine bessere Adaptierbarkeit der Seitenteile an ihre Funktionsaufgabe, beispielsweise die bessere Adaptierbarkeit an den von ihnen abzudichtenden Spalt aufgrund des Vorsehens von zwei Seitenteilen anstelle von nur einem, wie dies im Stand der Technik üblich ist. Zudem zeichnet sich die erfindungsgemäße Brille durch eine vereinfachte Adaptierbarkeit an unterschiedliche Gesichtsphysiognomien an. Ein Vorteil hierbei besteht in dem aufgrund der Trennung der Seitenteile erhöhten Tragekomfort. Ferner wird die zuverlässige Abdichtung des Spalts besser gewährleistet, falls dies die Aufgabe des Funktionsabschnittes ist. Zusätzlich können die Seitenteile kleiner, schmaler ausgestaltet werden und nehmen weniger Stauraum in ihrem Nichtbenutzungszustand, z. B. in der Hemdtasche, ein. Zudem kann die Schutzbrille mit einem optischen Glas beziehungsweise einer Sichtkorrektur ausgestattet werden und als einzige Brille des Trägers als Korrektur- und wahlweise auch als Schutzbrille ganztägig in seinem Arbeitsumfeld getragen werden. Das Mitführen von einer zweiten Brille kann entfallen.

Vorteilhafte Weiterbildungen des erfindungsgemäßen Gegenstands sind Gegenstand der Unteransprüche.

So wird in einer erfindungsgemäß bevorzugten Ausführungsform vorgeschlagen, dass das Rahmenteil der Brille zwei getrennt voneinander vorliegende Brillengläser mit einer zwischen den Brillengläsern angeordneten Brücke aufweist. Die Brücke kann dabei derart flexibel ausgestaltet sein, dass sie eine Anpassung der Brille an verschiedene Gesichtphysiognomien erlaubt. Hierzu kann die Brücke aus Polyamid, insbesondere einem thermoplastischen Kunststoff, hergestellt sein. Insbesondere kann sie ausgestaltet sein, um eine Winkelanpassung zwischen 30 und 55 Grad, insbesondere bezogen auf eine Stellung von zwei in je einer der beiden Scheibenebenen liegenden Geraden zueinander mit einem virtuellen Schnittpunkt vor der Brücke, ganz insbesondere bei der Projektion der Geraden auf eine Ebene senkrecht zu wenigstens einer Scheibenebene oder auf eine horizontale Ebene bei getragener Brille, zu erlauben.

Das Unterteilen der Sichtscheibe in zwei Brillengläser erlaubt eine wiederum bessere Anpassung der Brille an die Gesichtskrümmung eines Menschen allgemein, da die Brille mit einer größeren Gesamtkrümmung fertigbar und verwendbar ist, weshalb der verbleibende Spalt, den es zwischen Brille und Gesicht abzudecken gilt, schmaler ausfällt. Zudem kann die Brücke gemessen am Brillenglas bei der für sie geforderten Festigkeit flexibler ausgestaltet werden. Dies ermöglicht, dass die Brille unter Ausnutzung dieser Flexibilität bereits derart getragen werden kann, dass der Spalt zwischen Brille und Gesicht wiederum weiter verringert ist. Dies allein erhöht schon den Schutz für den Brillenträger vor einer Verletzung oder Irritation seiner Augen durch schädliche Stoffe oder Strahlungen und dergleichen aufgrund der verschmälerten Eintrittspforte. Zudem können die lösbar aufnehmbaren Seitenteile zur Erzielung derselben Abdichtwirkung des Spaltes wiederum kleiner ausgestaltet werden. Sie können somit noch einfacher, weniger störend und optisch unauffälliger vom Träger der Brille zu Zeiten, zu welchen sie zum Schutz der Augen nicht benötigt werden, beispielsweise in der Hemdtasche mitgeführt werden.

Die Brücke kann dabei derart ausgestaltet sein, dass sie vom Träger der Brille auf einfache Weise anpassbar ausgestaltet ist. Dies ist sie beispielsweise dann, wenn die Brücke ohne zu Hilfenahme von Werkzeug verformbar ist. Diese Verformbarkeit kann durch geeignete Materialwahl, aber auch durch geeignete geometrische Ausgestaltung der Brücke erzielt werden.

In einer wiederum weiter bevorzugten Ausführungsform ist die erste Halteeinrichtung und/ oder eine zweite Halteeinrichtung zum lösbaren Sichern der Brille am Kopf beispielsweise als ein Gummiband- oder eine Gummikordel derart ausgestaltet, dass sie um den Hinterkopf, Nacken oder Hals des Trägers herum die Brille sichert. Dabei kann ein Zug oder eine Vorspannung auf die Brille derart in Richtung auf die Augen des Brillenträgers hin ausgeübt werden, dass der Spalt zwischen Brille und Gesicht wiederum weiter verringert wird. Zudem erhöht die Halteeinrichtung der Brille dieser Ausführungsform die Sicherheit gegen ein unbeabsichtigtes Verlieren oder Herunterfallen der Brille. Letzteres kann insbesondere bei Arbeitsschutzbrillen im Zusammenhang mit der körperlichen Betätigung oder einfachen Bewegungen wie einem Bücken zum Aufheben eines Gegenstands vom Boden erforderlich sein.

Das Vorsehen einer Halteeinrichtung dieser Ausführungsform kann den Brillenträger ferner vorteilhaft davon abhalten, die Brille in Momenten, in welchen er sie nicht zum Schutz benötigt und er sie, da sie ihn stört, gerne absetzen möchte, nicht einfach abzulegen, um sie dann am abgelegten Ort zu vergessen. Vielmehr erlaubt die umgreifende Halteeinrichtung, die Brille auch im abgesetzten Zustand um den Hals baumelnd weiter mit sich zu tragen. Sie ist damit jederzeit verfügbar mitgeführt.

Bei einer wiederum weiter bevorzugten erfindungsgemäßen Ausführungsform sind die beiden Seitenteile mittels der Aufnahmeeinrichtung an wenigstens einem Abschnitt der Brille aufsteckbar. Durch das Vorsehen einer Aufsteckeinrichtung werden Nachteile des Standes der Technik -bei welchem beispielsweise das innere Rahmenteil der oben genannten WO 02/074209 A1, bei welcher ein Klickverschluss vorliegt - vorteilhaft vermieden. Ein Klickverschluss kann aufgrund von Verschleiß nicht dauerhaft zuverlässig bedienbar sein. Zudem konzentriert sich die Krafteinwirkung beim Einstecken und Ausstecken des inneren Rahmenteils des Standes der Technik auf eben die Strukturen des Klickverschlusses. Mechanische Überlastungen durch häufiges Ein- und Ausbauen des inneren Rahmenteils sowie durch unsachgemäße Benutzung sind im Stand der Technik gang und gäbe. Das Aufstecken der Seitenteile in der vorliegenden Ausführungsform der Erfindung hilft unter anderem diesen Nachteilen ab. Das Aufstecken kann durch eine einfache Anschlagwirkung z. B. eines Randes des Rahmenteils bewirkt werden, gegen welchen die Seitenteile z. B. durch die umgreifende Halteeinrichtung mit oder ohne Vorspannung gehalten werden. Das Aufstecken kann jedoch auch eine Passung zwischen Seitenteil und Brille bzw. Rahmenteil umfassen. Auch ein Reibschluss ist von der Erfindung umfasst.

Die Seitenteile können dabei derart ausgestaltet sein, dass sie aufgrund eines erhöhten oder ausgezogenen Randes den Spalt zwischen Brille und Gesicht beim Gebrauch umlaufend verringern oder gar ganz abdecken.

In einer weiter bevorzugten Ausführungsform ist das wenigstens eine Seitenteil und der zugehörige Funktionsabschnitt einstückig ausgestaltet, vorzugsweise beide aus demselben Material.

In einer weiter bevorzugten Ausführungsform sind das wenigstens eine Seitenteil und der zugehörige Funktionsabschnitt getrennt voneinander ausgestaltet. Sie können nach ihrer Herstellung miteinander verbunden worden sein, beispielsweise durch eine Klebeverbindung, müssen aber nicht. Aufgrund ihrer getrennten Herstellung können sie vorteilhaft unter geringem Herstellstellaufwand aus verschiedenen Materialien hergestellt sein. Dies erlaubt eine vorteilhafte Kombination verschiedener Materialien. So kann das Seitenteil, welches ggf. in das Rahmenteil eingesteckt werden und hierzu vorzugsweise eine ausreichende Festigkeit aufweisen soll, aus einem anderen Material gefertigt sein als der Funktionsabschnitt, welcher sich ggf. an das Gesicht des Brillenträgers anschmiegen soll ohne diesen zu stören und deshalb aus einem elastischeren, weicheren Material gefertigt sein könnte.

In einer wiederum bevorzugten Ausführungsform weist das Rahmenteil und/ oder die Seitenteile eine oder mehrere Belüftungseinrichtungen auf. Diese können als Belüftungsschlitze, -öffnungen oder dergleichen ausgestaltet sein. Sie können ferner mit einer geeigneten Filtereinrichtung zum Verhindern des Eintretens von Schadstoffen oder Strahlung bei gleichzeitiger klimatischer Wirkung ausgestaltet sein.

Die Brille kann in einer Zweikomponententechnik hergestellt sein. Dabei können der Rahmen und die Seitenteile aus geeigneten thermoplastischen Kunststoffen ausgestaltet sein. Damit ist die erforderliche Festigkeit der Brille durch das Rahmenteil gewährleistet, wohingegen die Seitenteile aus einem Material gefertigt sind, welches selbst vom Träger der Brille nachträglich noch auf einfache Weise mit Haushaltsmitteln auf seine besondere Passform angepasst werden kann. Die Brücke - sofern vorgesehen - kann wiederum aus einem anderen Material als das Rahmenteil hergestellt sein, z. B. einem Polyamid anderer Festigkeit.

In einer weiter bevorzugten Ausführungsform ist eine Nut vorgesehen, in welche die Brillengläser an Abschnitten ihres Randes aufgenommen werden können. Diese Nut kann umlaufend um die Öffnung des Brillenglases vorgesehen sein, sie kann jedoch auch nur an Abschnitten hiervon ausgestaltet sein. Die Nut kann einen beim Einsetzen des jeweiligen Brillenglases zu überwindenden Widerstand bieten und - nach Einsetzen des Brillenglases - dieses vor einem unbeabsichtigten Lösen aus dem Rahmenteil bzw. der Fassung bewahren. Durch das erforderliche Überwinden des Widerstandes beim Einsetzen eines Austauschbrillenglases durch den Träger der Brille, welcher in aller Regel kein Fachmann im Umgang mit Brillen und dem Austauschen von Brillengläsern ist, erhält man eine taktile Rückmeldung, ob das Brillenglas auch ausreichend tief in die Nut hineingedrückt wurde und ob das Brillenglas im Rahmenteil zuverlässig fest eingesetzt ist.

Eine umlaufende Lippe kann in räumlicher Nähe zur oben beschriebenen Nut vorgesehen sein, gegen welche das Brillenglas bei seinem Einsetzen unter Kraftaufwand gedrückt werden kann. Die Lippe stellt sicher, dass auch bei Drücken des Brillenglases in das Rahmenteil hinein das Brillenglas nicht über die Nut hinweg auf der gegenüberliegenden Seite wieder aus dem Rahmenteil hinausgedrückt werden kann. Umgekehrt kann durch das Vorsehen der Lippe sichergestellt werden, dass das Brillenglas z. B. bei einem Scheibenwechsel wegen Verschmutzung oder Verkratzen sicher in das Rahmenteil hineingedrückt wird: Das Brillenglas ist lediglich mit mehr oder weniger Kraft bis gegen die Lippe zu drücken. Die Lippe kann umlaufend oder abschnittsweise ausgestaltet sein.

In einer weiter bevorzugten Ausführungsform ist das wenigstens eine Seitenteil vornehmlich zum Verringern oder vollständigen Verschlusses des Spaltes zwischen Rahmenteil und Gesicht des Trägers der Brille im Gebrauch ausgestaltet. Die mittels der Brille in dieser erfindungsgemäßen Ausführungsform erzielbaren Vorteile umfassen - insbesondere bei Vorsehen von zwei getrennten Seitenteilen - unter anderem eine bessere Adaptierbarkeit der Seitenteile an den von ihnen abzudichtenden Spalt aufgrund des Vorsehens von zwei Seitenteilen anstelle von nur einem, wie dies im Stand der Technik üblich ist. Zudem zeichnet sich die erfindungsgemäße Brille durch eine vereinfachte Adaptierbarkeit an unterschiedliche Gesichtsphysiognomien an. Ein Vorteil hierbei besteht in dem aufgrund der Trennung der Seitenteile erhöhten Tragekomfort. Ferner wird die zuverlässige Abdichtung des Spalts besser gewährleistet. Zusätzlich können die Seitenteile kleiner, schmaler ausgestaltet werden und nehmen weniger Stauraum in ihrem Nichtbenutzungszustand, z. B. in der Hemdtasche, ein. Zudem kann die Schutzbrille mit einem optischen Glas beziehungsweise einer Sichtkorrektur ausgestattet werden und als einzige Brille des Trägers als Korrektur- und wahlweise auch als Schutzbrille ganztägig in seinem Arbeitsumfeld getragen werden. Das Mitführen von einer zweiten Brille kann entfallen.

In einer weiter bevorzugten Ausführungsform weist die Brille wenigstens ein mit einem ringförmigen Abschnitt zum Verbinden des Seitenteils mit dem Rahmenteil oder einem anderen Abschnitt der Brille ausgestaltetes Seitenteil auf. Dieser ringförmige Abschnitt - welcher keine vollständig umlaufende bzw. geschlossene Form aufweisen muss - kann ähnlich einer Scheibe mit innen liegender Öffnung vergleichsweise flach und daher mit vergleichsweise großer Auflagefläche - gemessen an seiner Bautiefe - ausgestaltet sein. Er kann, wie in einer weiter bevorzugten Ausführungsform der Erfindung, mittels der Aufnahmeeinrichtung an der Brille bzw. dem Rahmenteil oder einem anderen Abschnitt der Brille angeordnet werden. Dabei können jeweils ein Seitenteil und wenigstens ein ringförmiger Abschnitt einstückig hergestellt werden, was den Herstellaufwand und die hiermit verbundenen Kosten senkt.

Ein weiterer, mit dem Vorsehen des ringförmigen Abschnitts verbundener Vorteil besteht darin, dass die konstruktive Ausgestaltung der Aufnahmeeinrichtung als Verbindungseinrichtung zwischen Brille und Seitenteil bspw. über verschiedene Seitenteile hinweg stets gleich gefertigt werden kann, wobei sich verschiedene Seitenteile im wesentlichen nur im Aufbau des Funktionsabschnitts unterscheiden. Dadurch lässt sich der Herstellaufwand für die Brille deutlich senken. Es ist ferner sichergestellt, dass unterschiedliche Seitenteile und vor allem Funktionsabschnitte mit ein und derselben Brille zuverlässig verwendbar sind.

Die Aufnahmeeinrichtung kann dabei derart ausgestaltet sein, dass sie eine spannungsarme oder spannungsfreie Aufnahme des wenigstens einen ringförmigen Abschnitts im Einbauzustand erlaubt. Dies kann bspw. mittels eines Verriegelns - und ggf. eines Einsteckens an einer zweiten, ggf. gegenüberliegenden Seite - des ringförmigen Abschnitts erfolgen. Dazu kann, wie in einer weiteren Ausführungsform vorgesehen, die Halteeinrichtung von einer ersten Stellung, in welcher sie ein Lösen des ringförmigen Abschnitts vom Rahmenteil bzw. Brille allgemein nicht verhindert, in eine zweite Stellung, in welcher sie ein Lösen des ringförmigen Abschnitts vom Rahmenteil verhindert, überfiihrbar - bspw. verschwenkbar, einrastbar, klappbar, verschiebbar oder dergleichen mehr - sein. Dabei kann beispielsweise - ohne hierauf beschränkt zu sein - die Halteeinrichtung den ringförmigen Abschnitt in der zweiten Stellung mittels eines Vorsprungs der Halteinrichtung gegen ein unbeabsichtigtes Lösen vom Rahmenteil bewahren. Die Halteeinrichtung kann wiederum durch eine Reibeinrichtung, eine Einrasteinrichtung oder dergleichen in der beabsichtigten - bspw. der zweiten - Stellung gehalten werden. Dies erlaubt vorteilhaft ein ermüdungsfreies Einsetzen und Herausnehmen der Seitenteile in die bzw. aus der Brille. Ferner ist ein leichteres Reinigen und Austauschen der Seitenteile möglich. Die Bereitschaft, die Seitenteile zum Schutz bei Bedarf auch wirklich zu verwenden, steigt ferner an, da hierzu keine besondere handwerkliche Geschicklichkeit erforderlich ist und zudem vom Brillenträger nicht befürchtet werden muss, dass die Seitenteile durch den Befestigungsvorgang beschädigt oder ermüdet werden.

Die Erfindung wird im Folgenden anhand einer möglichen beispielhaften Ausführungsform unter Bezugnahme auf die beigefügte Zeichnung detaillierter erläutert. In der Zeichnung gilt:
- Fig. 1: zeigt die erfindungsgemäße Brille ohne aufgesteckte Seitenteile;
- Fig. 2: zeigt zwei Seitenteile;
- Fig. 3: zeigt die Brille der Fig. 1 mit aufgesteckten Seitenteilen;
- Fig. 4: zeigt ein Brillenglas der erfindungsgemäßen Brille in schematisch vereinfachter Darstellung; und
- Fig. 5: zeigt eine erfindungsgemäße Brille mit einem Seitenteil mit ringförmigem Abschnitt;
- Fig. 6: zeigt die Brille der Fig. 5 in einem teilweise demontierten Zustand in einer Ansicht von einer Augenseite des Patienten aus;
- Fig. 7: zeigt die Brille der Fig. 5 und 6 in vergrößerter, ausschnittweiser Darstellung in Blickrichtung des Brillenträgers (d. h. von innen bzw. von der Augenseite);
- Fig. 8: zeigt die Brille der Fig. 5 bis 7 in einem Ausschnitt, wobei die erste Halteeinrichtung in einer ersten Stellung steht; und
- Fig. 9: zeigt die Brille der Fig. 8 in einer zweiten Stellung der ersten Halteeinrichtung.

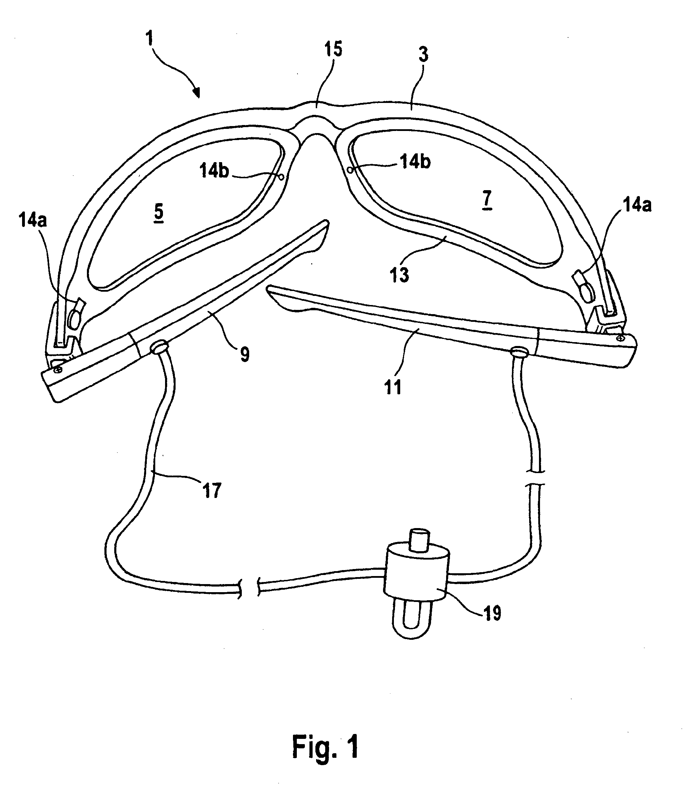

Fig. 1 zeigt eine erfindungsgemäße Brille 1 einer erfindungsgemäßen Ausführungsform. Die Brille 1 weist ein Rahmenteil 3 mit zwei Brillengläsern 5 und 7 und zwei klappbaren oder wahlweise nicht klappbaren Brillenbügeln 9 und 11 als Halteeinrichtungen zum Halten der Brille 1 am Kopf eines Trägers auf. Die Brille 1 weist an ihrem Rahmenteil 3 ferner einen als Aufnahmeeinrichtung für die Brillengläser 5 und 7 ausgestalteten Rand auf. Dieser Rand 13 ist in der Darstellung der Fig. 1 umlaufend, d. h. geschlossen, ausgestaltet. Er kann einem Unterfüttern der Brillengläser 5 und 7 im Rahmenteil 3 dienen. Er kann dies im Sinne eines Anschlags sein.

Die Brille 1 weist ferner eine Einschiebeöffnung 14a sowie eine Einstecköffnung 14b auf, mittels welcher sie vorbereitet ist, um weiter unten beschriebene Seitenteile auf- und absteckbar aufzunehmen.

Die Brille 1 der Fig. 1 weist eine zwischen den Brillengläsern 5 und 7 angeordnete Brücke 15 auf, welche im Ausführungsbeispiel der Fig. 1 einstückig mit dem Rahmenteil 3 hergestellt ist. Einstückigkeit ist jedoch nicht erforderlich. Die Brücke 15 und das Rahmenteil 3 können auch zusammensteckbar gefertigt sein. Die Brücke 15 ermöglicht eine Unterteilung der Brillengläser 5 und 7 in zwei getrennt voneinander vorliegende Brillengläser. Sie kann, wie in der Ausführungsform der Fig. 1 gezeigt, ferner eine Abstützung des Rahmenteils 3 oder eines anderen Abschnittes der Brille 1 auf dem Nasenrücken des Trägers der Brille 1 ermöglichen oder begünstigen. Die Brücke 15 kann dabei derart flexibel ausgestaltet sein, dass die Brille 1 im Bereich der Brücke 15 reversibel und zerstörungsfrei an die spezielle Gesichtsphysiognomie des Brillenträgers anpassbar ist. Dies kann durch Auswahl entsprechender Materialien und/ oder Ausgestaltung der Brücke 15 mit geeigneter Geometrie bewirkt werden. Besonders bevorzugt ist die Brücke 15 derart flexibel ausgestaltet, dass das Rahmenteil 3 durch Zug mittels einer um den Hinterkopf des Brillenträgers gelegten Halteeinrichtung 17 in Form eines mittels Justiereinrichtung 19 in seiner Länge veränderbaren Bands der Gesichtskontur des Brillenträgers weiter anpassbar ist. Die Halteeinrichtung 17 kann hierbei an jedem Abschnitt der Brille 1 angeordnet sein. Sie kann ferner als die einzige Halteeinrichtung vorgesehen sein und damit der ersten Halteeinrichtung entsprechen.

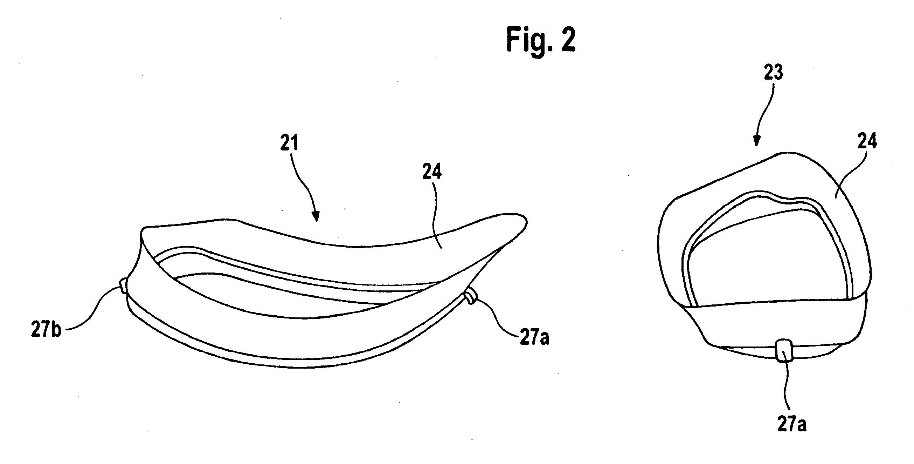

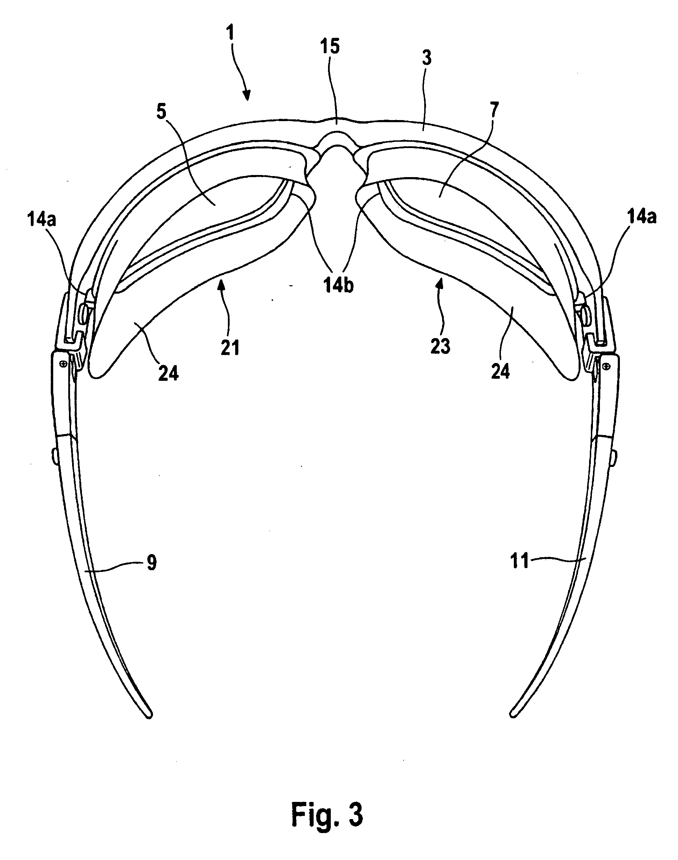

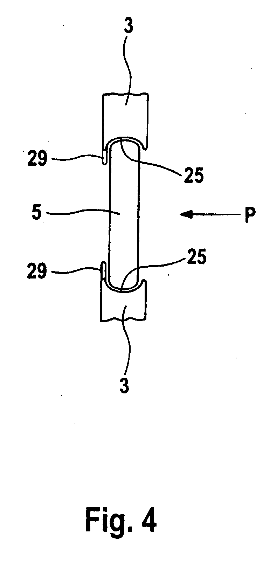

Fig. 2 zeigt Seitenteile 21 und 23, welche, wie in Fig. 3 gezeigt ist, mit der Brille 1 der Fig. 1 abnehmbar verbindbar sind. Die Seitenteile 21 und 23 sind aus einem tragefreundlichen Material gefertigt, welches ggf. auch durch nachträgliche Bearbeitung wie Zuschneiden ein über die fabrikseitig vorgenommene Adaption hinaus zusätzliches Anpassen der Brille 1 an die Gesichtsoberfläche im Augenbereich erlaubt. Zu erkennen sind in Fig. 2 ein überhöhter bzw. hoher Rand 24 der Seitenteile 21, 23, welcher sich bis an die Haut des Trägers heran erstreckt. Eine Einschiebelippe 27a kann den Seitenteilen 21, 23 durch ihr Einschieben in die Einschiebeöffnung 14a gemeinsam mit einem Einsteckstift 27b zum Einstecken in die Einstecköffnung 14b zu einem festeren Sitz im Rahmenteil 3 verhelfen. Jedoch sind Einrichtungen wie die Einschiebelippen 27a oder die Einsteckstifte 27b entbehrlich. Es können alternative Verbindungen vorgesehen sein. Die Seitenteile 21, 23 können ganz oder im Wesentlichen durch einen auf das Rahmenteil 3 in Richtung auf die Augen des Brillenträgers aufgebrachten Zug am Rahmenteil 3 gehalten werden. Die Einrichtungen 27a und 27b können allein zum Sichern der Seitenteile 21 und 23 gegen ein Verrutschen derselben bezogen auf das Rahmenteil 3 vorgesehen sein. Damit tragen sie vorteilhaft zur Erhöhung der Zuverlässigkeit des Augenschutzes bei. Dabei sind sie mechanisch nicht beansprucht. Ein Verschleiß tritt somit nicht auf. Da die Seitenteile mittels der Einrichtungen 27a und 27b derart mühelos auf- und abzusetzen sind, wird vom Brillenträger hiervon auch Gebrauch gemacht. Der Fall, in welchem der Brillenträger die Seitenteile nicht anbringt - obwohl erforderlich - um sich ein mühevolles Einsetzen der Seitenteile zu ersparen, tritt bei dem erfindungsgemäßen System der Fig.4 sowie in seiner Verallgemeinerung vorteilhaft nicht auf. Die Einschiebelippe 27a kann dabei ebenso wie der Einsteckstift 27b vorgesehen sein, um ohne Kraftaufwand in die entsprechenden Öffnungen 14a und 14b einzubringbar zu sein. Die Einrichtungen 27 können zum Charakter der erfindungsgemäßen Brille als eine Wechselbrille beitragen.

Es wird angemerkt, dass es sich bei den in den Figuren dargestellten Einrichtungen 14a, b und 27a, b um beispielhafte Ausführungen der Aufnahmeeinrichtungen und Aufnahmen handelt. Selbstverständlich können auch zwei oder mehr Einschiebstifte vorgesehen sein bei Verzicht auf Einschiebstifte oder umgekehrt. Auch andere Kombinationen sind möglich. Die Stifte können als Vorsprünge oder Haken jeder geeigneten Art ausgestaltet sein, bspw. als Nase und Pin. Das Einsetzen der Einrichtungen 27 kann durch ein Einstecken des Einsteckstifts 27b, gefolgt vom Einschieben der Einschiebelippe 27a erfolgen. Zum Abnehmen der Seitenteile 21, 23 wird in umgekehrter Reihenfolge vorgegangen. Auch ein Anbringen der Seitenteile 21, 23 am Rahmenteil 3 in nur einer Bewegungsrichtung kann durch Vorsehen entsprechend - ein, zwei oder mehr- ausgestalteter Einrichtungen 27 vorgesehen sein.

Die Öffnungen 14a und 14b können als Durchgangsöffnungen ausgestaltet sein, sie können jedoch auch als Sacklöcher ausgestaltet sein. Die Öffnungen können als Vertiefungen oder Ösen jeder geeigneten Art ausgestaltet sein.

Fig. 3 zeigt die Brille der Fig. 1 ohne die dort dargestellte bandförmige Halteeinrichtung 17 mit den in der Fig. 2 dargestellten Seitenteilen 21 und 23 in einem Schutzzustand, d. h. mit aufgesteckten Seitenteilen. Gut zu entnehmen ist der Fig. 3, dass die Seitenteile 21 und 23 aufgrund der Krümmung des Rahmenteils 3, aber auch aufgrund des erhöhten Randes 24 der Seitenteile 21 und 23 zum Kontaktieren einer bogenförmigen Gesichtsstruktur des Trägers der Brille 1 ausgestaltet sind.

Die Brille 1 kann mit in der Zeichnung nicht dargestellten Belüfiungsöffnungen oder -schlitzen ausgestaltet sein, welche beispielsweise oberhalb der Brillengläser 5 und 7 im Rahmenteil 3 angeordnet sind. Diese Belüftungseinrichtung kann jedoch auch in, sowie zusätzlich in, den Seitenteilen 21 und 23 vorgesehen sein. Zum Verhindern des Eintretens von schädlichen Substanzen oder Strahlungen oder dergleichen durch die Belüftungseinrichtung in einen Raum zwischen den Brillengläsern und den Augen des Trägers kann beispielsweise eine Filtereinrichtung, eine geeignete Membran oder dergleichen vorgesehen sein.

Fig. 4 zeigt schematisch vereinfacht einen Schnitt durch das Brillenglas 5, welches in seinem oberen und seinem unteren Bereich in einer Nut 25, welche Teil des in Fig. 4 nicht dargestellten Rahmenteils ist, gehalten wird. Die Nut 25 dient dabei dem Halten des Brillenglases 5 im Rahmenteil. Zudem gibt die Nut 25 aufgrund ihres Widerstands beim Scheibenwechsel dem Benutzer eine Auskunft darüber, ob das Brillenglas korrekt eingesetzt ist.

In Fig. 4 ist ferner eine Lippe 29 dargestellt, welche ebenfalls Teil des nicht dargestellten Rahmenteils ist. Gegen diese Lippe 29 kann das Brillenglas 5 in Richtung des Pfeils P bis zum Einrasten in die Nut 25 gedrückt werden ohne Gefahr zu laufen, dass das Brillenglas 5 auf der dem Pfeil P abgewandten Seite wieder aus der Nut 25 herausgedrückt wird. Die Lippe 29 kann die Funktion des oben diskutierten Rands 13 ganz oder teilweise übernehmen.

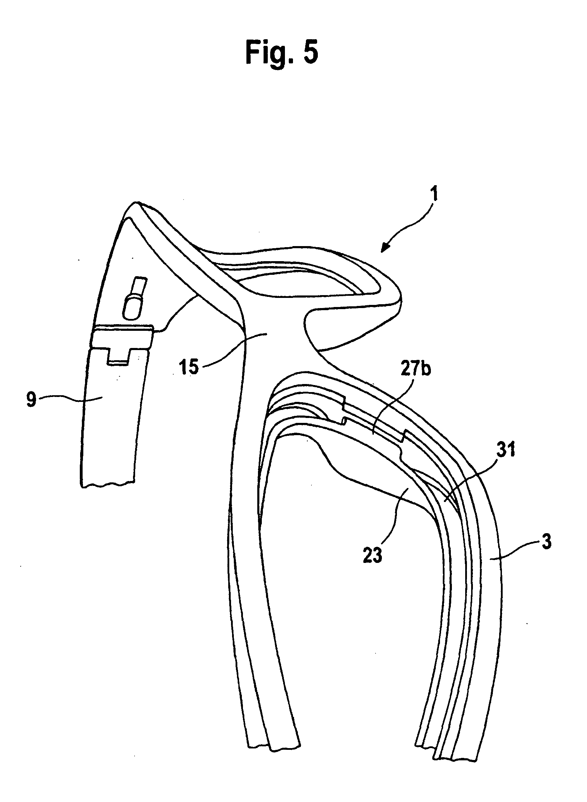

Fig. 5 zeigt in Teildarstellung eine erfindungsgemäße Brille 1 mit einem Seitenteil 23 mit ringförmigem Abschnitt 31 beim noch nicht abgeschlossenen Vorgang des Einsteckens des Seitenteils 23 in das Rahmenteil 3. Der ringförmige Abschnitt 31 kann bei jeder erfindungsgemäßen Ausführungsform - wie in Fig. 5 exemplarisch gezeigt - dieselbe Krümmung, Form, Biegung oder dergl. wie ein Abschnitt des Rahmenteils 3 aufweisen, so dass sich der ringförmige Abschnitt 31 und das Rahmenteil 3 bei Anfügen des Seitenteils 23 eng aneinander anlegen. Letzteres trägt vorteilhaft zu einem besseren Abschluss von Seitenteil 23 und Rahmenteil 3 bei und kann eine Abdichtung zwischen Seitenteil 23 und Rahmenteil 3 bewirken.

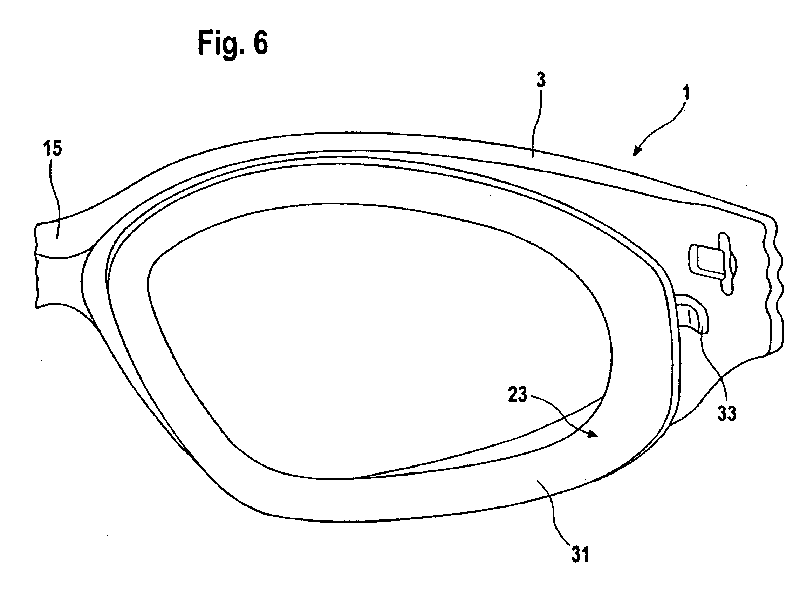

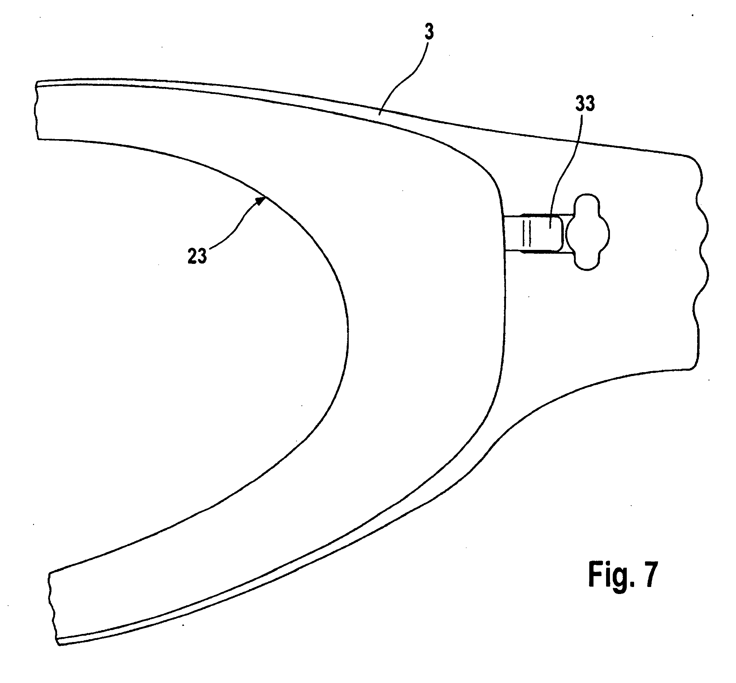

Fig. 6 zeigt die Brille 1 der Fig. 5 in einem teilweise demontierten Zustand in einer Ansicht von einer Augenseite des Patienten aus. Dabei ist der ringförmige Abschnitt 31 getrennt von seinem Funktionsabschnitt (der Funktionsabschnitt ist in Fig. 6 nicht dargestellt) gezeigt. Das Seitenteil 23 trägt dabei eine Nase 33, welche im Zusammenspiel mit einem in Fig. 7 nicht dargestellten Vorsprung ein Lösen des Seitenteils 23 vom Rahmenteil 3 vorteilhaft ohne nennenswerte mechanische oder andere Belastung für das Seitenteil 23 verhindern kann.

Fig. 7 zeigt wiederum die Brille 1 der Fig. 5 und 6 in vergrößerter, ausschnittsweiser Darstellung in Blickrichtung des Brillenträgers (d. h. von innen bzw. von der Augenseite).

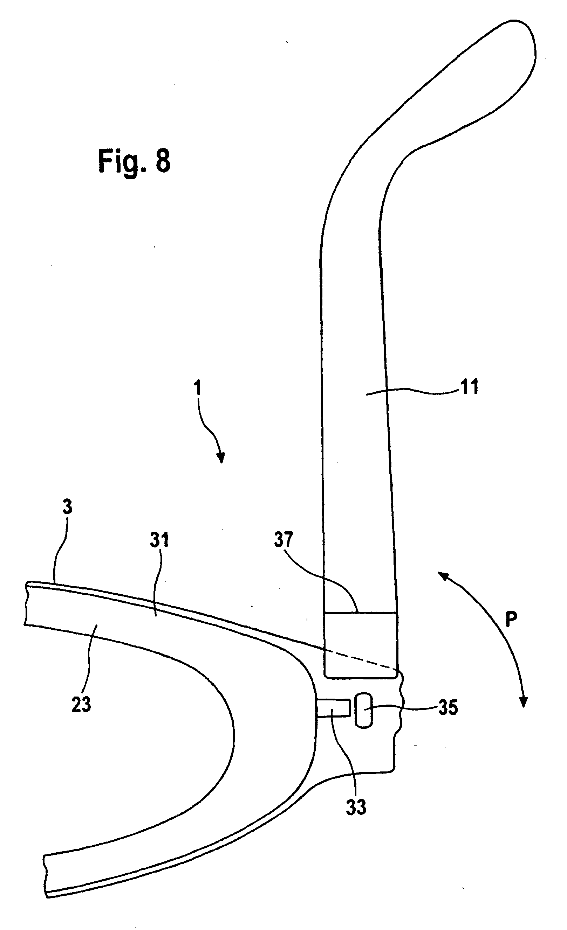

Fig. 8 zeigt die Brille 1 der Fig. 5 bis 7 in einem Ausschnitt. Dabei steht die erste - hier als Brillenbügel ausgestaltete - Halteeinrichtung 11 in einer ersten Stellung, in welcher sie ein Lösen des an seinem linken Rand (nicht dargestellt in Fig. 8) in das Rahmenteil 3 oder die Brücke 15 oder einen anderen Brillenabschnitt eingeschobenen, eingesteckten oder dgl. Seitenteils 23 bzw. dessen ringförmigen Abschnitts 31 nicht verhindert. Ein solches Lösen könnte verhindert werden durch den Vorsprung 35 der Halteinrichtung 11, welcher zu diesem Zweck vor (bzw. in Blickrichtung des Brillenträgers dem Auge näher) verschwenkt wird und ein Herausfallen des Seitenteils in Richtung Auge verhindert. Eine solche Verschwenkbewegung ist bspw. entlang des Pfeils P der Fig. 8 möglich. Es wird angemerkt, dass diese Verschwenkbewegung nicht mit einem Ein- und Ausklappen der Halteeinrichtung 11 beim Auf- oder Absetzen der Brille um ein Scharnier 37 zu verwechseln ist. Zwar können beide zuletzt genannten Bewegungen kombiniert werden, was erfindungsgemäß auch angedacht ist, jedoch ist dies erfindungsgemäß nicht erforderlich.

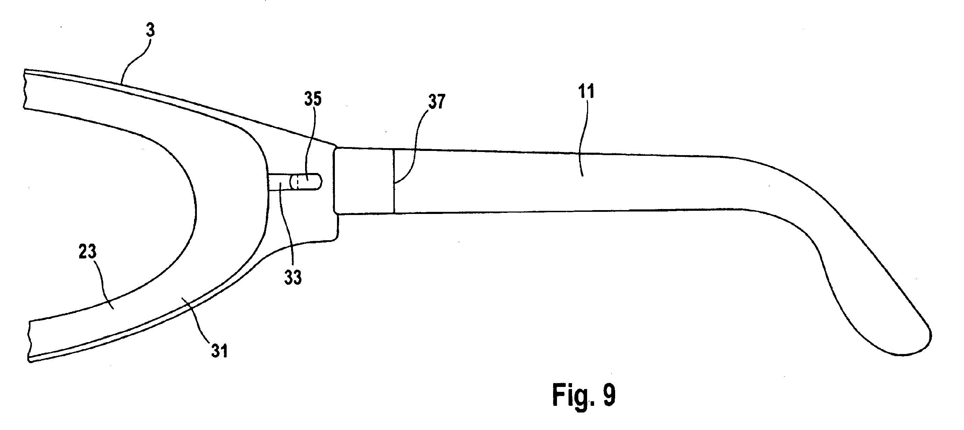

Fig. 9 zeigt die Brille der Fig. 8 in der zweiten Stellung der ersten Halteeinrichtung 11, in welcher der mit dieser verschwenkte Vorsprung 35 die Nase 33 derart überdeckt, dass ein Herausfallen des Seitenteils 23 bzw. seines ringförmigen Abschnitts 31 aus dem Rahmenteil 3 verhindert ist.

## Patentansprüche

1. Brille (I), insbesondere Schutzbrille, mit wenigstens einem Rahmenteil (3) mit wenigstens einem Brillenglas (5, 7) und wenigstens einer ersten Halteeinrichtung (9, 11) zum Halten der Brille (I) am Kopf eines Trägers mit wenigstens einer Aufnahmeeinrichtung (14a, 14b), welche ein Seitenteil oder zwei getrennt voneinander vorliegende Seitenteile (21, 23) lösbar aufnimmt, wobei die Brille (1) die Seitenteile (21, 23) aufweist, wobei wenigstens ein Seitenteil (21, 23) einen Funktionsabschnitt zum Schutz der Augen und/ oder zum Verändern der Funktion des Brillenglases (5, 7) aufweist, wobei wenigstens ein Seitenteil (21, 23) einen ringförmigen Abschnitt (31) zum Verbinden des Seitenteils (21, 23) mit dem Rahmenteil (3) und/ oder mit einem anderen Abschnitt der Brille (1) aufweist, **dadurch gekennzeichnet, dass** die erste Halteeinrichtung (9, 11), welche einen Vorsprung (35) aufweist, von einer ersten Stellung, in welcher sie ein Lösen des ringförmigen Abschnitts (31) vom Rahmenteil (3) mittels des Vorsprungs (35) nicht verhindert, in eine zweite Stellung, in welcher sie ein Lösen des ringförmigen Abschnitts (31) vom Rahmenteil (3) mittels des Vorsprungs (35) verhindert, überführbar ist.

2. Brille (1) nach Anspruch 1, **dadurch gekennzeichnet, dass** das Rahmenteil (3) zwei getrennt voneinander vorliegende Brillengläser (5, 7) aufnimmt und eine zwischen den Brillengläsern (5, 7) angeordnete Brücke (15) aufweist.

3. Brille (1) nach Anspruch 2, **dadurch gekennzeichnet, dass** die Brücke (15) an die Gesichtsphysiognomie des Trägers der Brille (1) von diesem anpassbar ausgestaltet ist.

4. Brille (1) nach einem der vorangegangenen Ansprüche, **dadurch gekennzeichnet, dass** die beiden Seitenteile (21, 23) mittels der Aufnahmeeinrichtung (14a, 14b) an der Brille (1), insbesondere an deren Rahmenteil (3), aufsteckbar sind.

5. Brille (1) nach einem der vorangegangenen Ansprüche, bei welcher das Rahmenteil (3) und/ oder die Seitenteile (21, 23) einen oder mehrere thermoplastische Kunststoffe aufweisen.

6. Brille (1) nach einem der vorangegangenen Ansprüche, **gekennzeichnet durch** einen Rand (13), insbesondere eine Nut (25) zum Begrenzen des Brillenglases (5, 7).

7. Brille (1) nach einem der vorangegangenen Ansprüche, **gekennzeichnet durch** eine Lippe (29) zum Erleichtern eines Wechselns des Brillenglases (5, 7).

8. Brille (1) nach einem der vorangegangenen Ansprüche, **dadurch gekennzeichnet, dass** das wenigstens eine Seitenteil oder beide Seitenteile (21, 23) mit einem Funktionsabschnitt zum Verringern eines Spalts zwischen Rahmenteil (3) und Gesicht des Trägers der Brille (1) im Gebrauch ausgestaltet sind.

9. Brille (1) nach einem der vorangegangenen Ansprüche, **dadurch gekennzeichnet, dass** die Aufnahmeeinrichtung (14a, 14b, 27a, 27b) zum Verbinden des Rahmenteils (3) oder eines anderen Abschnitts der Brille (1) mit dem ringförmigen Abschnitt (31) des wenigstens einen Seitenteils (21, 23) ausgestaltet ist.

10. Brille (1) nach einem der vorangegangenen Ansprüche, **dadurch gekennzeichnet, dass** wenigstens ein Seitenteil (23) und wenigstens ein ringförmiger Abschnitt (31) einstückig hergestellt sind.

11. Brille (1) nach einem der vorangegangenen Ansprüche, **dadurch gekennzeichnet, dass** die Aufnahmeeinrichtung (14a, 14b) für eine spannungsarme oder spannungsfreie Aufnahme des wenigstens einen ringförmigen Abschnitts (31) im Einbauzustand ausgestaltet ist.

12. Brille (1) nach einem der vorangegangenen Ansprüche, wobei die Halteeinrichtung (9, 11) den ringförmigen Abschnitt (31) in der zweiten Stellung mittels eines Vorsprungs (35) der Halteinrichtung (9, 11) gegen ein unbeabsichtigtes Lösen vom Rahmenteil (3) bewahrt.

13. Seitenteile (21, 23) zur Verwendung mit der Brille (1) nach einem der vorangegangenen Ansprüche, wobei die Seitenteile (21, 23) ausgestaltet sind wie in einem der vorangegangenen Ansprüche definiert.

14. Set mit einer Brille (1) nach einem der Ansprüche 1 bis 13 und voneinander verschiedenen, alternativ zu verwendenden Seitenteilen (21, 23), wobei die Seitenteile (21, 23) entsprechend der jeweiligen Anwendung unterschiedlich ausgestaltet sind, wobei die jeweiligen ringförmigen Abschnitte (31) identisch ausgeführt sind.

## Claims

1. Eyeglasses (1), particularly safety glasses, having at least one frame part (3) with at least one eyeglass lens (5, 7) and at least a first retaining means (9, 11) for retaining the eyeglasses (1) on the head of a wearer with at least one receiving means (14a, 14b) for detachably receiving one side part or two discrete side parts (21, 23), wherein the eyeglasses (1) comprise the side parts (21, 23), wherein at least one side part (21, 23) has a functional section for protecting the eyes and/or for changing the function of the eyeglass lens (5, 7), wherein at least one side part (21, 23) has an annular section (31) for connecting the side part (21, 23) with the frame part (3) and/or with any other section of the eyeglasses (1), **characterized in that** the first retaining means (9, 11), which includes a protrusion (35), can be transferred from a first position in which it does not prevent detachment of the annular section (31) from the frame part (3) by means of the protrusion (35), to a second position in which it prevents detachment of the annular section (31) from the part part (3) by means of the protrusion (35).

2. The eyeglasses (1) according to claim 1, **characterized in that** the frame part (3) receives two discrete eyeglass lenses (5, 7) and includes a bridge (15) interposed between the eyeglass lenses (5, 7).

3. The eyeglasses (1) according to claim 2, **characterized in that** the bridge (15) is configured so as to be adaptable by the wearer of the eyeglasses (1) to the facial physiognomy of the wearer of the eyeglasses (1).

4. The eyeglasses (1) according to any of the preceding claims, **characterized in that** the two side parts (21, 23) can be attached to the eyeglasses (1), particularly to the frame part (3) thereof, by means of the receiving means (14a, 14b).

5. The eyeglasses (1) according to any of the preceding claims, wherein the frame part (3) and/or the side parts (21, 23) comprise one or more thermoplastics.

6. The eyeglasses (1) according to any of the preceding claims, **characterized by** a rim (13), particularly a groove (25), for delimiting the eyeglass lens (5, 7).

7. The eyeglasses (1) according to any of the preceding claims, **characterized by** a lip (29) for facilitating change of the eyeglass lens (5, 7).

8. The eyeglasses (1) according to any of the preceding claims, **characterized in that** the at least one side side part or both side parts (21, 23) are configured with a functional section for reducing a gap between the frame part (3) and the face of the wearer of the eyeglasses (1) during use.

9. The eyeglasses (1) according to any of the preceding claims, **characterized in that** the receiving means (14a, 14b, 27a, 27b) is configured to connect the frame part (3) or any other section of the eyeglasses (1) with the annular section (31) of the at least one side part (21, 23).

10. The eyeglasses (1) according to any of the preceding claims, **characterized in that** at least one side part (23) and at least one annular section (31) are made from one piece.

11. The eyeglasses (1) according to any of the preceding claims, **characterized in that** the receiving means (14a, 14b) is configured to receive the at least one annular section (31) in a low-tension or tension-free manner while mounted.

12. The eyeglasses (1) according to any of the preceding claims, wherein the retaining means (9, 11) in the second position protects the annular section (31) by means of a protrusion (35) of the retaining means (9, 11) from inadvertent detachment from the frame part (3).

13. Side parts (21, 23) for use with the eyeglasses (1) according to any of the preceding claims, wherein the side parts (21, 23) are configured as defined in any of the preceding claims.

14. A kit comprising eyeglasses (1) according to any of claims 1 to 13 and mutually different side parts (21, 23) to be used alternatively, wherein the side parts (21, 23) are configured differently so as to match the respective application and wherein the respective annular sections (31) are configured identically.

## Revendications

1. Lunettes (1), en particulier, lunettes de protection, comportant au moins un élément de monture (3) doté au moins d'un verre de lunettes (5, 7) et d'au moins un premier dispositif de retenue (9, 11) pour retenir les lunettes (1) sur la tête d'un porteur, comportant au moins un dispositif de réception (14a, 14b) qui reçoit de façon détachable une partie latérale ou deux parties latérales (21, 23) séparées l'une de l'autre, les lunettes (1) présentant les parties latérales (21, 23), dans lesquelles au moins une partie latérale (21, 23) présente un segment de fonction pour la protection des yeux et/ou pour la modification de la fonction des verres de lunettes (5, 7), au moins une partie latérale (21, 23) présentant un segment circulaire (31) pour relier la partie latérale (21, 23) avec l'élément de monture (3) et/ou avec un autre segment des lunettes (1), **caractérisées en ce que** le premier dispositif de retenue (9, 11) qui présentent une proéminence (35), peut passer d'une première position dans laquelle il n'empêche pas un détachement du segment circulaire (31) de la monture (3) au moyen de la proéminence (35) à une deuxième position dans laquelle il empêche un détachement du segment circulaire (31) de l'élément de monture (3) au moyen de la proéminence (35).

2. Lunettes (1) selon la revendication 1, **caractérisées en ce que** l'élément de monture (3) reçoit deux verres de lunette (5, 7) séparés l'un de l'autre et un pont (15) disposé entre les verres de lunette (5, 7).

3. Lunettes (1) selon la revendication 2, **caractérisé en ce que** le pont (15) est conçu de façon à s'adapter à la physionomie du visage du porteur de lunettes (1) par celui-ci.

4. Lunettes (1) selon l'une des revendications précédentes, **caractérisées en ce que** les deux parties latérales (21, 23) peuvent être emboîtées au moyen d'un dispositif de réception (14a, 14b) sur les lunettes (1), en particulier sur leur élément de monture (3).

5. Lunettes (1) selon l'une des revendications précédentes, dans lesquelles l'élément de cadre (3) et/ou les parties latérales (21, 23) présentent une ou plusieurs matières plastiques thermoplastiques.

6. Lunettes (1) selon l'une des revendications précédentes, **caractérisées par** un bord (13), en particulier, une rainure (25) pour limiter les verres de lunettes (5, 7)

7. Lunettes (1) selon l'une des revendications précédentes, **caractérisées par** une lèvre (29) pour faciliter un changement des verres de lunettes (5, 7).

8. Lunettes (1) selon l'une des revendications précédentes, **caractérisées en ce qu'**au moins une partie latérale ou les deux parties latérales (21, 23) sont conçues avec un segment de fonction pour réduire un espace entre l'élément de monture (3) et le visage du porteur de lunettes (1) lors de l'utilisation.

9. Lunettes (1) selon l'une des revendications précédentes, **caractérisées en ce que** le dispositif de réception (14a, 14b, 27a, 27b) est conçu pour relier l'élément de monture (3) ou un autre segment des lunettes (1) avec le segment circulaire (31) d'au moins une partie latérale (21, 23).

10. Lunettes (1) selon l'une des revendications précédentes, **caractérisé en ce qu'**au moins une partie latérale (23) et au moins un segment circulaire (31) sont fabriqués d'un seul tenant.

11. Lunettes (1) selon l'une des revendications précédentes, **caractérisées en ce que** le dispositif de réception (14a, 14b) est conçu pour une réception à faible tension ou sans tension d'au moins un segment circulaire (31) à l'état monté.

12. Lunettes (1) selon l'une des revendications précédentes, dans lesquelles le dispositif de retenue (9, 11) prévient un détachement involontaire du segment circulaire (31) dans la deuxième position, de l'élément de monture (3), au moyen d'une proéminence (35) du dispositif de retenue (9, 11).

13. Parties latérales (21, 23) pour l'utilisation avec les lunettes (1) selon l'une des revendications précédentes, les parties latérales (21, 23) étant conçues comme défini dans l'une des revendications précédentes.

14. Ensemble comportant des lunettes (1) selon l'une des revendications 1 à 13, et des parties latérales (21, 23) différentes l'une de l'autre, à utiliser alternativement, les parties latérales (21, 23) étant conçues différemment en fonction de l'utilisation respective, les segments circulaires respectifs (31) étant identiques.
